# EUROPEAN PATENT SPECIFICATION

(11) **EP 2 823 844 B1**
(45) Date of publication and mention of the grant of the patent: **15.08.2018**
(21) Application number: 13757687.2
(22) Date of filing: 28.02.2013
(51) Int. Cl.: A61M 16/12, A61M 16/00, A61M 16/04, A61M 16/14, A61M 16/10

(54) **ARTIFICIAL RESPIRATOR**
KÜNSTLICHE BEATMUNGSVORRICHTUNG
APPAREIL DE RESPIRATION ARTIFICIELLE

(30) Priority: 09.03.2012 JP 2012053018
(43) Date of publication of application: 14.01.2015
(73) Proprietor: Air Water Inc., Sapporo-shi Hokkaido 060-0003 (JP)
(72) Inventor: AIKAWA, Tetsuya, Matsumoto-shi Nagano 390-1701 (JP); BABA, Atsushi, Matsumoto-shi Nagano 390-0877 (JP)
(74) Representative: Boult Wade Tennant LLP
(86) International application number: PCT/JP2013/055360
(87) International publication number: WO 2013/133117

(56) References cited:
- WO-A1-2007/006377
- WO-A2-2011/073839
- JP-A- 2000 279 521
- JP-A- 2001 517 108
- JP-A- 2010 220 711
- JP-U- H0 595 549
- US-A1- 2005 005 936
- US-A1- 2009 107 503
- US-A1- 2010 212 668

## Description

### TECHNICAL FIELD

The present invention relates to a ventilator for supplying a mixed gas of oxygen and a medical gas other than oxygen to a patient as an inhalation gas.

### BACKGROUND ART

In an ordinary (artificial) ventilation therapy, ventilation is performed 15 to 20 times per minute with a tidal volume of 6 to 10 mL/kg of body weight. For example, in the case of an adult with a body weight of 60 kg, the tidal volume is 360 to 600 mL. This volume is larger than the volume of an anatomical dead space (that is, the nasal cavity and trachea/bronchi which are not involved in gas exchange between oxygen and carbon dioxide) of a human, and allows a fresh gas to be transported sufficiently into the lung alveoli.

However, in such an (artificial) ventilation therapy, the gas is forced into the lungs with pressure, unlike human natural breathing. Accordingly, the lungs expand and contract to a large degree, causing cracks in the connection between the cells of the lungs. Thus, a disorder called Ventilator Induced Lung Injury (VILI) may occur.

As an (artificial) ventilation therapy developed to prevent this VILI, a ventilation method called High Frequency Oscillation (HFO) is provided, as disclosed in Japanese Utility Model Laying-Open No. 58-16146 (PTD 1). The HFO is a ventilation method for performing ventilation with a tidal volume smaller than the volume of the anatomical dead space, at a ventilation frequency of several to more than 10 Hz. The HFO urges gas exchange by means of oscillations, so to speak, by minutely vibrating the lungs. Thus, the lungs hardly expand and contract and the possibility of occurrence of the VILI is reduced, unlike the ordinary (artificial) ventilation therapy described above.

On the other hand, for patients with respiratory failure having airway stenosis and the like, provided is a treatment method of causing a patient to inhale a mixed gas of helium and oxygen, instead of a mixed gas of air and oxygen, as a gas used for the (artificial) ventilation therapy (hereinafter referred to as a "helium and oxygen inhalation therapy") in addition to the HFO, as disclosed in Andrew Katz et al., "Heliox Improves Gas Exchange during High-frequency Ventilation in a Pediatric Model of Acute Lung Injury", Am. J. Respir. Crit. Care Med., Jul 15; 164(2), pp. 260 to 264, (2001) (NPD 1), and Bakhtiyar Zeynalov et al., "Effects of heliox as carrier gas on ventilation and oxygenation in an animal model of piston-type HFOV: a crossover experimental study.", Biomed. Eng. Online., Nov 12; 9:71 (2010) (NPD 2). It is noted that the mixed gas of helium and oxygen may be referred to as "heliox" in Europe and the United States.

In the helium and oxygen inhalation therapy described above, since helium has a low density, the gas easily flows through even a narrow airway, and it is expected that ventilation is improved even in the patients having airway stenosis. Irrespective of the ordinary ventilator or the HFO ventilator, the helium and oxygen inhalation therapy can be performed by supplying the mixed gas of helium and oxygen to a compressed gas inlet of the ventilator, and filling the ventilator and the entire respiratory circuit with the mixed gas of helium and oxygen.

The ordinary (artificial) ventilation therapy and the HFO described above are performed by inserting and placing a tube called an intratracheal tube in a trachea of a patient. Generally, the intratracheal tube is composed of one tube. In this case, however, gas exchange has a poor efficiency because inhalation gas and exhalation gas of the patient pass through the same tube. Therefore, an intratracheal tube having a plurality of tubes is also considered, as disclosed in Japanese Patent National Publication No. 2009-504240 (PTD 2), Japanese Patent Laying-Open No. 2008-93328 (PTD 3), and Japanese Patent National Publication No. 2002-524155 (PTD 4). In the intratracheal tube having the plurality of tubes, some tubes are used to supply an inhalation gas to a patient, and the remaining tubes are used to emit an exhalation gas. Such a ventilation method is disclosed in Avi Nahum, "Equipment review: Tracheal gas insufflation", Crit. Care., 2(2), pp. 43 to 47, (1998) (NPD 3), and is called Tracheal Gas Insufflation (TGI).

### CITATION LIST

### PATENT DOCUMENT

PTD 1: Japanese Utility Model Laying-Open No. 58-16146
PTD 2 : Japanese Patent National Publication No. 2009-504240
PTD 3 : Japanese Patent Laying-Open No. 2008-93328
PTD 4 : Japanese Patent National Publication No. 2002-524155

### NON PATENT DOCUMENT

NPD 1 : Andrew Katz et al., "Heliox Improves Gas Exchange during High-frequency Ventilation in a Pediatric Model of Acute Lung Injury", Am. J. Respir. Crit. Care Med., Jul 15; 164(2), pp. 260 to 264, (2001)
NPD 2 : Bakhtiyar Zeynalov et al., "Effects of heliox as carrier gas on ventilation and oxygenation in an animal model of piston-type HFOV: a crossover experimental study.", Biomed. Eng. Online., Nov 12; 9:71(2010)
NPD 3 : Avi Nahum, "Equipment review: Tracheal gas insufflation", Crit. Care., 2(2), pp. 43 to 47, (1998)

### SUMMARY OF INVENTION

### TECHNICAL PROBLEM

However, when the helium and oxygen inhalation therapy is performed using the method disclosed in NPD 2, a gas at about 8 L/min as a gas passing through a respiratory circuit, and a gas at about 28 L/min for driving the HFO ventilator, that is, gases at about 36 L/min in total are consumed, as described later in experimental examples. To comply with the regulation on high-pressure gas, helium is often supplied in a cylinder having a capacity of 7000 L, and one cylinder is consumed in about 194 minutes. A patient who needs the helium and oxygen inhalation therapy often requires gas inhalation continuously for several days, and in such a case, the cylinders should be replaced frequently. Further, there has been a problem that this work places an increasing burden on medical staff. In addition, there has also been a problem that, since helium is a rare natural resource, it is expensive, and since helium is consumed in a large amount, the cost required for treatment is also increased.

Also in the ordinary ventilator other than the HFO ventilator, gases other than a gas inhaled by a patient are consumed, such as a gas passing through a respiratory circuit, and a gas used for driving the ventilator. Thus, although the helium and oxygen inhalation therapy is considered as an effective method for patients from the viewpoint of treatment, it has been difficult to commonly use the therapy because the amount of gas used poses significant practical and economic problems.

The present invention has been made to solve the aforementioned problems, and one object of the present invention is to provide a ventilator capable of performing effective (artificial) ventilation while reducing a consumption amount of a medical gas other than oxygen.

### SOLUTION TO PROBLEM

The present invention relates to a ventilator for supplying a mixed gas of oxygen and a medical gas other than oxygen to a patient as an inhalation gas, including a first gas supply device for supplying a gas containing oxygen, wherein the first gas supply device (2) supplies the gas containing oxygen by high frequency oscillation; an intratracheal tube path inserted and placed in a trachea of the patient, an extratracheal tube path for connecting the first gas supply device with the intratracheal tube path and guiding the gas containing oxygen from the first gas supply device to the patient, and a second gas supply device connected to the extratracheal tube path or the intratracheal tube path for supplying the mixed gas of oxygen and the medical gas other than oxygen to the extratracheal tube path or the intratracheal tube path.

Preferably, in the ventilator in accordance with the present invention, the medical gas other than oxygen is helium.

Preferably, in the ventilator in accordance with the present invention, a flow meter for measuring a flow rate of the mixed gas of oxygen and the medical gas other than oxygen is connected between the second gas supply device and the intratracheal tube path or the extratracheal tube path.

Preferably, in the ventilator in accordance with the present invention, an oxygen concentration monitor for measuring an oxygen concentration in the mixed gas of oxygen and the medical gas other than oxygen is connected between the second gas supply device and the intratracheal tube path or the extratracheal tube path.

Preferably, in the ventilator in accordance with the present invention, an oxygen concentration monitor for measuring an oxygen concentration in the gas containing oxygen on a ventilator side from a location where the second gas supply device is connected is connected between the extratracheal tube path and the first gas supply device.

Preferably, in the ventilator in accordance with the present invention, the intratracheal tube path has two independent flow paths, and is configured such that the gas containing oxygen from the first gas supply device flows through one flow path, and the mixed gas of oxygen and the medical gas other than oxygen from the second gas supply device flows through the other flow path.

Preferably, in the ventilator in accordance with the present invention, the gas containing oxygen from the first gas supply device contains compressed air.

Preferably, in the ventilator in accordance with the present invention, the first gas supply device is configured to emit an exhalation gas from the patient passing through the intratracheal tube path and the extratracheal tube path.

### ADVANTAGEOUS EFFECTS OF INVENTION

According to the present invention, a ventilator capable of performing effective (artificial) ventilation while reducing a consumption amount of a medical gas other than oxygen can be provided.

### BRIEF DESCRIPTION OF DRAWINGS

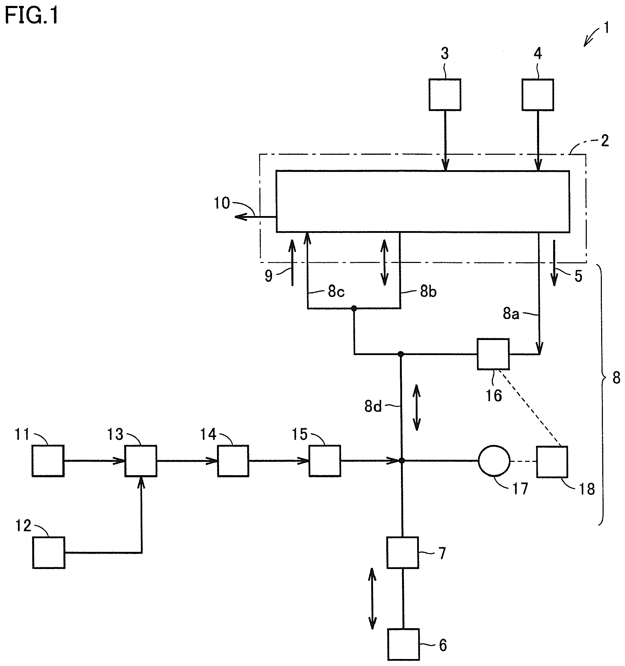

Fig. 1 is a view schematically showing a ventilator 1 in a preferred example of the present invention.

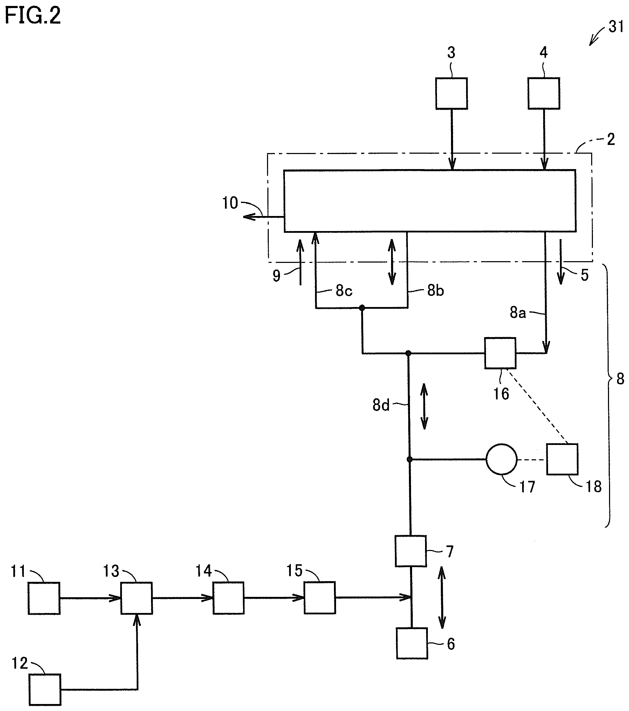

Fig. 2 is a view schematically showing a ventilator 31 in another preferred example of the present invention.

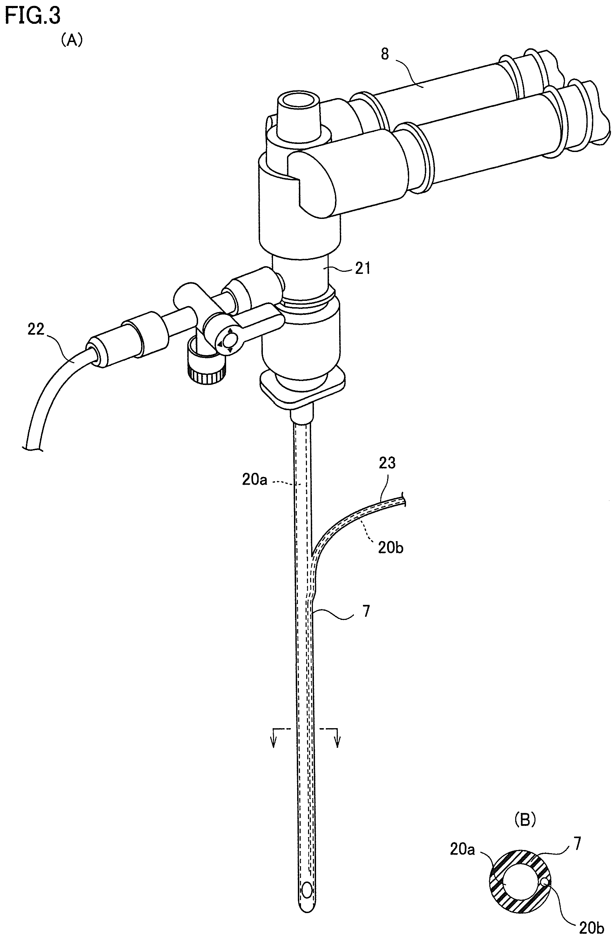

Fig. 3 is a view schematically showing an example of an extratracheal tube path and an intratracheal tube path suitably used for ventilator 31 shown in Fig. 2, in which Fig. 3(A) is a perspective view, and Fig. 3(B) is a cross sectional view thereof.

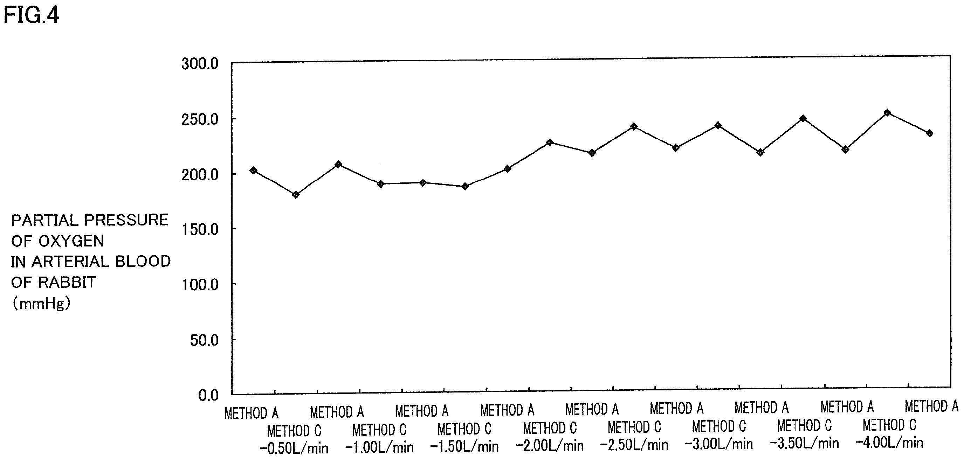

Fig. 4 is a graph showing changes in the partial pressure of oxygen in the arterial blood of a rabbit when an administration amount in method C is set in Experimental Example 2, in which the axis of ordinate represents the partial pressure of oxygen in the arterial blood of the rabbit (mmHg).

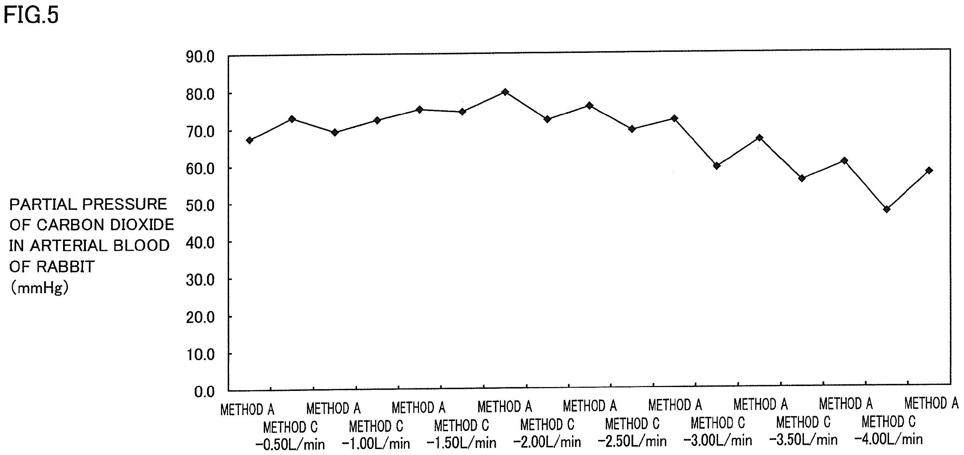

Fig. 5 is a graph showing changes in the partial pressure of carbon dioxide in the arterial blood of the rabbit when the administration amount in method C is set in Experimental Example 2, in which the axis of ordinate represents the partial pressure of carbon dioxide in the arterial blood of the rabbit (mmHg).

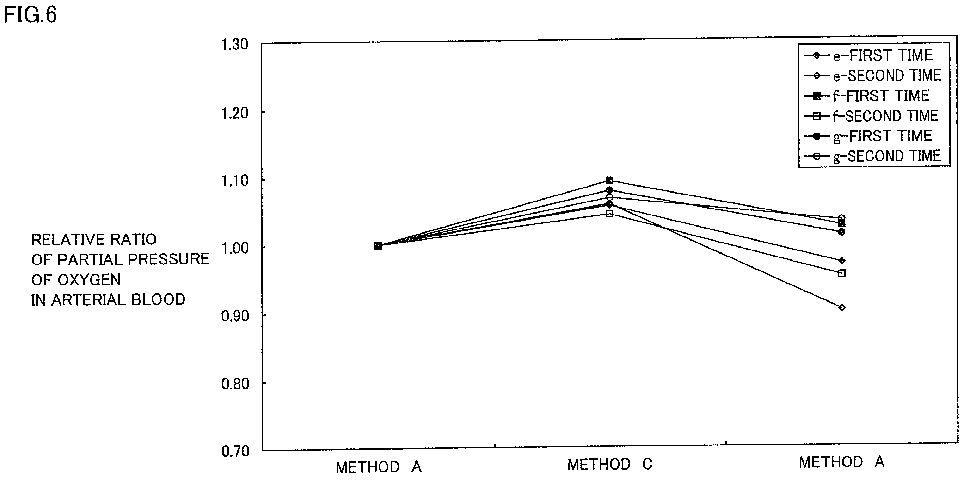

Fig. 6 is a graph showing relative changes in the partial pressure of oxygen in the arterial blood of rabbits when (artificial) ventilation is performed in the order of method A, method C, and method A in Experimental Example 2, in which the axis of ordinate represents the relative ratio of the partial pressure of oxygen in the arterial blood of the rabbits.

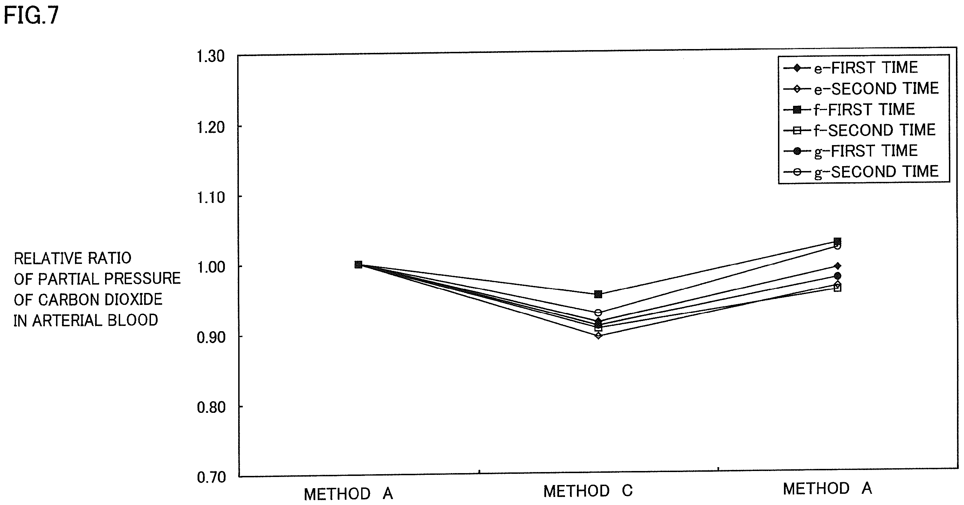

Fig. 7 is a graph showing relative changes in the partial pressure of carbon dioxide in the arterial blood of the rabbits when (artificial) ventilation is performed in the order of method A, method C, and method A in Experimental Example 2, in which the axis of ordinate represents the relative ratio of the partial pressure of carbon dioxide in the arterial blood of the rabbits.

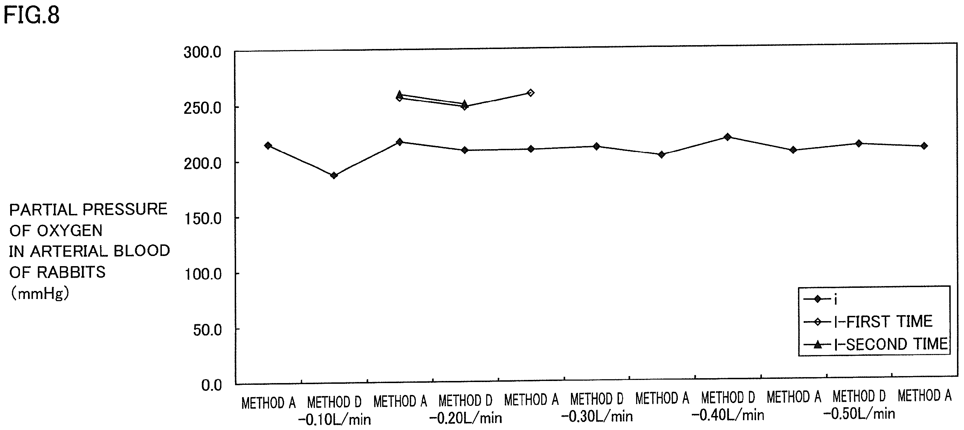

Fig. 8 is a graph showing changes in the partial pressure of oxygen in the arterial blood of rabbits when an administration amount in method D is set in Experimental Example 3, in which the axis of ordinate represents the partial pressure of oxygen in the arterial blood of the rabbits (mmHg).

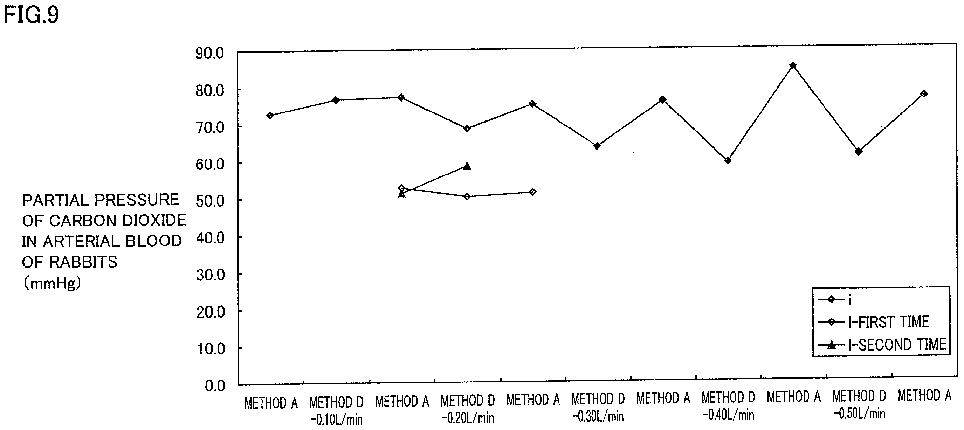

Fig. 9 is a graph showing changes in the partial pressure of carbon dioxide in the arterial blood of the rabbits when the administration amount in method D is set in Experimental Example 3, in which the axis of ordinate represents the partial pressure of carbon dioxide in the arterial blood of the rabbits (mmHg).

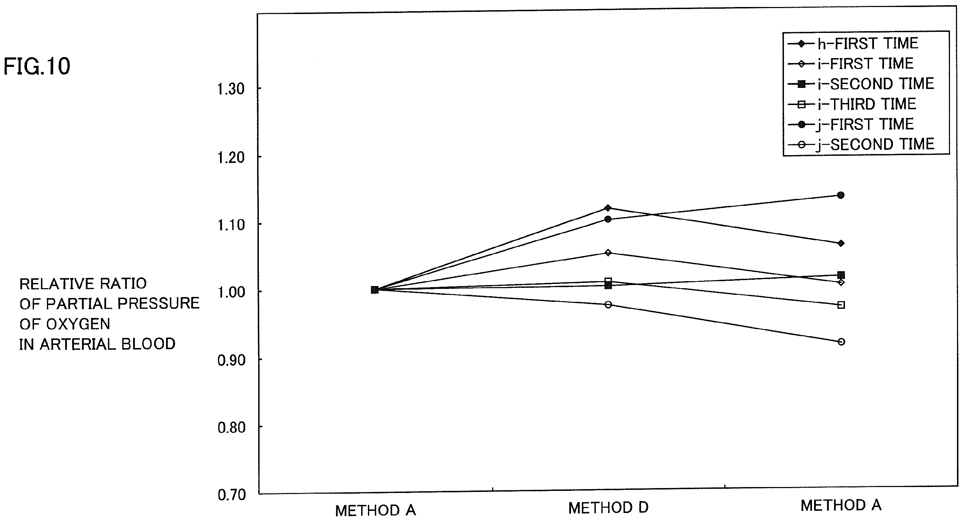

Fig. 10 is a graph showing relative changes in the partial pressure of oxygen in the arterial blood of rabbits when (artificial) ventilation is performed in the order of method A, method D, and method A in Experimental Example 3, in which the axis of ordinate represents the relative ratio of the partial pressure of oxygen in the arterial blood of the rabbits.

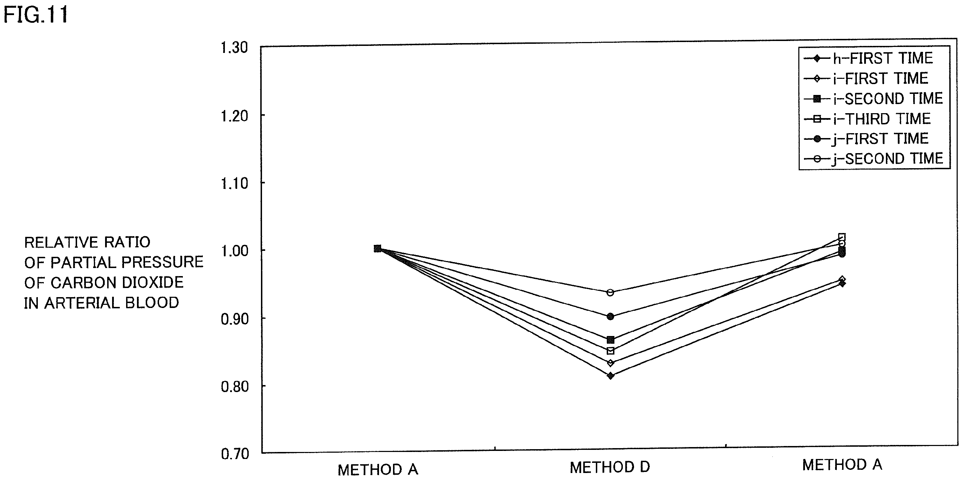

Fig. 11 is a graph showing relative changes in the partial pressure of carbon dioxide in the arterial blood of the rabbits when (artificial) ventilation is performed in the order of method A, method D, and method A in Experimental Example 3, in which the axis of ordinate represents the relative ratio of the partial pressure of carbon dioxide in the arterial blood of the rabbits.

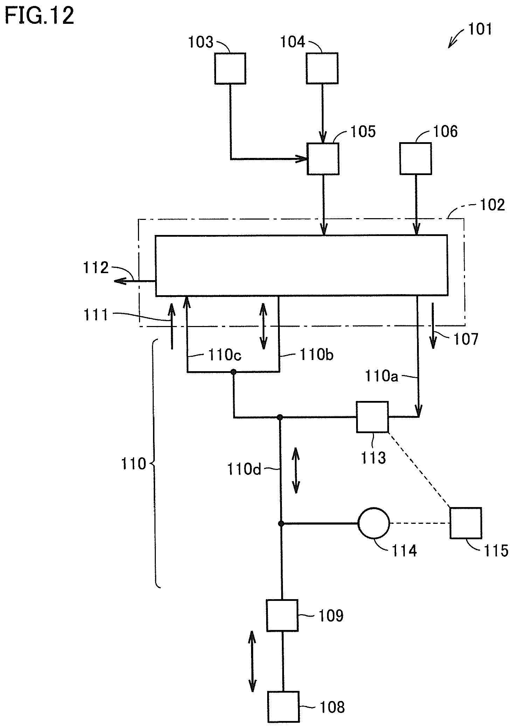

Fig. 12 is a view schematically showing a ventilator 101 in a case where the helium and oxygen inhalation therapy is performed using method A.

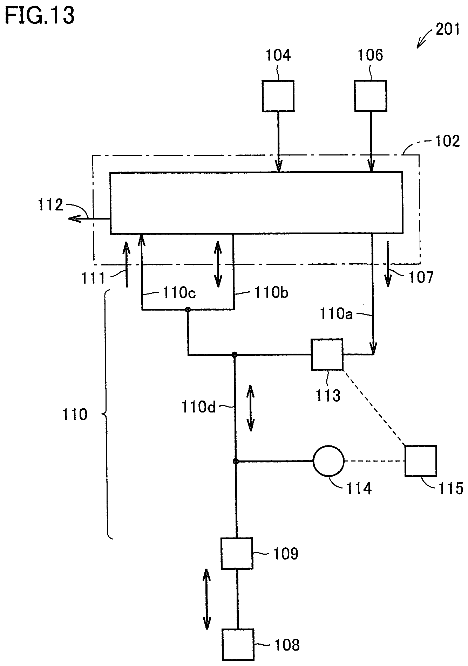

Fig. 13 is a view schematically showing a ventilator 201 in a case where it is used for method B.

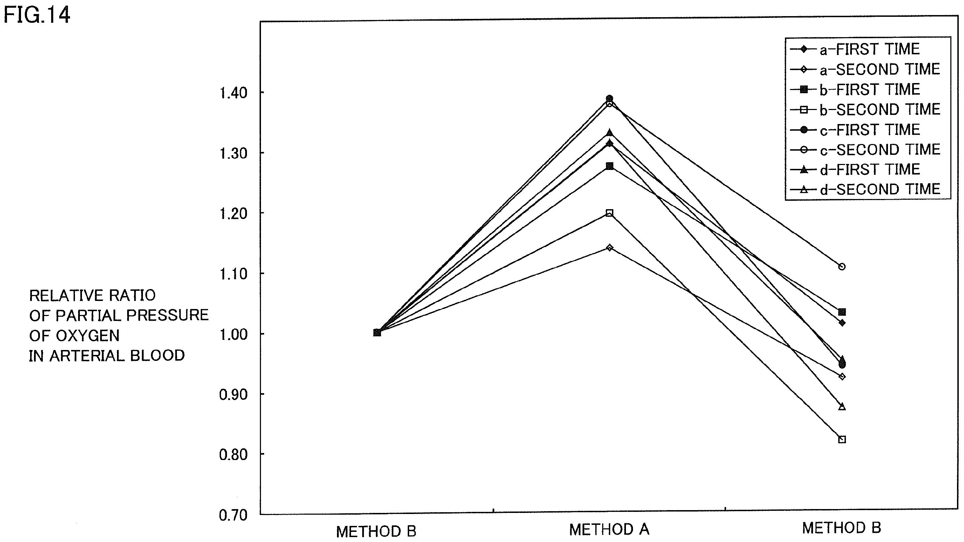

Fig. 14 is a graph showing relative changes in the partial pressure of oxygen in the arterial blood of rabbits when (artificial) ventilation is performed in the order of method B, method A, and method B in Experimental Example 1, in which the axis of ordinate represents the relative ratio of the partial pressure of oxygen in the arterial blood of the rabbits.

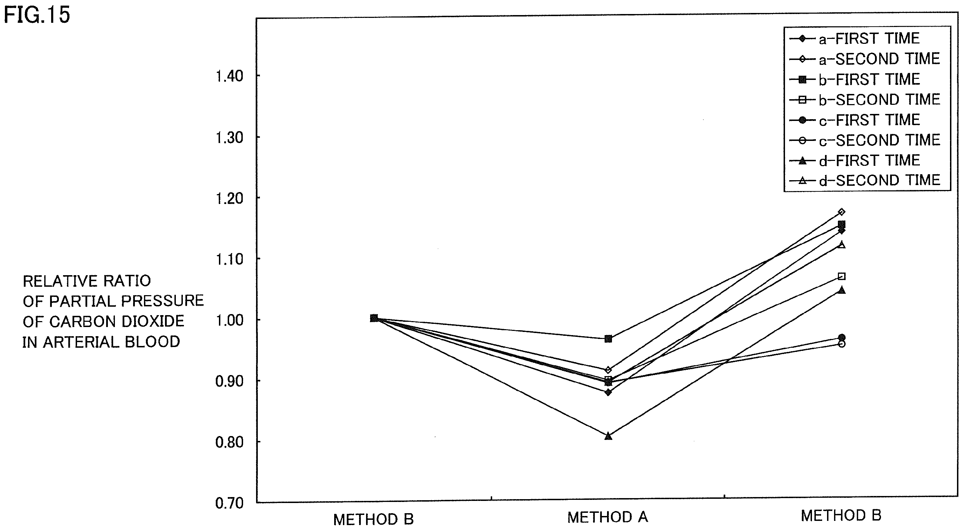

Fig. 15 is a graph showing relative changes in the partial pressure of carbon dioxide in the arterial blood of the rabbits when (artificial) ventilation is performed in the order of method B, method A, and method B in Experimental Example 1, in which the axis of ordinate represents the relative ratio of the partial pressure of carbon dioxide in the arterial blood of the rabbits.

### DESCRIPTION OF EMBODIMENTS

Fig. 1 is a view schematically showing a ventilator 1 in a preferred example of the present invention. Ventilator 1 in accordance with the present invention is characterized by including a first gas supply device (also referred to as a "main body" of the ventilator) 2 for supplying a gas containing oxygen by high frequency oscillation, an intratracheal tube path (intratracheal tube) 7 inserted and placed in a trachea of a patient 6, an extratracheal tube path 8 for connecting first gas supply device 2 with intratracheal tube path 7 and guiding the gas containing oxygen from first gas supply device 2 to patient 6, and a second gas supply device (mixer) 13 connected to extratracheal tube path 8 for supplying a mixed gas of oxygen and a medical gas other than oxygen to extratracheal tube path 8. It is noted that, in the ventilator in accordance with the present invention, a portion between an end portion of extratracheal tube path 8 connected to first gas supply device 2 and an end portion on the side close to first gas supply device 2 of intratracheal tube path 7 inserted and placed in the trachea of patient 6 will be referred to as a "respiratory circuit". Further, in extratracheal tube path 8, the side connected to first gas supply device 2 will be referred to as a "ventilator side", and the side connected to intratracheal tube path 7 will be referred to as a "patient side".

In the present invention, the "medical gas" refers to a gas which exhibits a desired medical effect without causing any damage to a human body even if it is administered to the human body in a medically acceptable range. While oxygen is also included in the medical gas, examples of the medical gas other than oxygen used in the present invention include helium, xenon, hydrogen, nitric oxide, carbon monoxide, hydrogen sulfide, nitrous oxide, carbon dioxide, nitrogen, argon, krypton, radon, and the like. Other examples include a volatile anesthetic such as sevoflurane, isoflurane, halothane, and desflurane, and an agent generally called a steroid inhaler. Among them, when helium is used as the medical gas other than oxygen, the effect that the gas easily flows through even a narrow airway and ventilation can be improved even in patients having airway stenosis is exhibited, as demonstrated in Experimental Example 1 described later. Further, when xenon, argon, krypton, and the volatile anesthetic are each used as the medical gas other than oxygen, effects such as anesthesia and neuronal protection are exhibited; when hydrogen is used, an effect such as neuronal protection is exhibited; when nitric oxide is used, effects such as dilatation of pulmonary vessels and anti-inflammation are exhibited; when carbon monoxide is used, an effect such as dilatation or constriction of pulmonary vessels is exhibited, depending on the administered concentration; when hydrogen sulfide is used, effects such as hibernation, organ protection, and anti-inflammation are exhibited; when nitrous oxide is used, an effect such as anesthesia is exhibited; when carbon dioxide is used, effects such as excitation of the respiratory center and dilatation of vessels are exhibited; when nitrogen is used, an effect such as an increase in the pulmonary vascular resistance is exhibited; when radon is used, an effect such as anti-inflammation is exhibited; and when the steroid inhaler is used, effects such as bronchodilation and anti-inflammation are exhibited.

According to the ventilator in accordance with the present invention, a consumption amount of the medical gas other than oxygen can be significantly reduced, when compared with the method of administering a mixed gas of oxygen and a medical gas other than oxygen by the HFO in the (artificial) ventilation therapy, as demonstrated in the experimental examples described later. Thus, the frequency of replacing cylinders of the medical gas other than oxygen during (artificial) ventilation can also be significantly reduced, leading to a reduction of the burden on medical staff. This is considered to be because, since the mixed gas of oxygen and the medical gas other than oxygen is not used as a gas for driving the ventilator, and the mixed gas of oxygen and the medical gas other than oxygen is supplied from the vicinity of the intratracheal tube path in the ventilator in accordance with the present invention, the consumption amount of the mixed gas of oxygen and the medical gas other than oxygen can be reduced without deteriorating the effect of improving the efficiency of (artificial) ventilation using the mixed gas of oxygen and the medical gas other than oxygen.

In the example shown in Fig. 1, oxygen 3 and compressed air 4 are supplied separately to main body 2 of ventilator 1. From main body 2, the gas containing oxygen (in the example shown in Fig. 1, oxygen 3 and compressed air 4) is supplied to the respiratory circuit as an inhalation gas 5. Preferably, main body 2 used for the present invention is configured to supply the gas containing oxygen by the high frequency oscillation (HFO), because the possibility of occurrence of the VILI can be reduced. As such main body 2, a commercially available device used for (artificial) ventilation can be used as appropriate, and for example, a piston-type HFO ventilator named Humming II (manufactured by S. K. I. Net, Inc.) or the like can be suitably used. Further, as oxygen 3, commercially available oxygen can be used without particular limitation, and specifically, Japanese Pharmacopoeia Oxygen (manufactured by Shinano Air Water Inc., purity: 99.5% or more) or the like can be suitably used.

In a case where inhalation gas 5 is a mixed gas of oxygen and compressed air, the mixing ratio therebetween is not particularly limited.

In ventilator 1 in the example shown in Fig. 1, extratracheal tube path 8 is branched at some locations partway therealong, and includes, other than a tube path 8a for allowing inhalation gas 5 described above to pass therethrough, a tube path 8b for transferring oscillations caused by the HFO from main body 2, a tube path 8c for returning an exhalation gas 9 from patient 6 to main body 2, and a tube path 8d having a patient side thereof connected to intratracheal tube path 7. In the example shown in Fig. 1, extratracheal tube path 8 is configured such that tube path 8b and tube path 8c join at a location partway therealong, further join tube path 8a on a patient side thereof, and connect to tube path 8d. Inhalation gas 5 passes through tube path 8a, then passes through tube path 8d, and flows to intratracheal tube path 7 together with the oscillations caused by the HFO transferred through tube path 8b. On the other hand, exhalation gas 9 from patient 6 passes through tube path 8d and tube path 8c and is returned to main body 2, and thereafter is emitted from main body 2 as an emission gas 10. Extratracheal tube path 8 can be composed for example by using commercially available airtight tubes, connection members, and the like as appropriate, and the branch of extratracheal tube path 8 can be formed for example by using a Y tube.

Ventilator 1 in the preferred example of the present invention is mainly characterized by including the second gas supply device for supplying the mixed gas of oxygen and the medical gas other than oxygen to extratracheal tube path 8. Fig. 1 shows an example in which ventilator 1 is configured such that helium 11 as an example of the medical gas other than oxygen is mixed with oxygen 12 by mixer 13 as the second gas supply device, the mixed gas of helium 11 and oxygen 12 is supplied from a location partway along extratracheal tube path 8, and is administered together with inhalation gas 5 through intratracheal tube path 7. As the second gas supply device, a commercially available device can be used without particular limitation, and for example, a gas mixer named He+O₂ Blender (manufactured by Air Water Inc.) or the like can be suitably used. Also, any commercially available oxygen and helium can be used, and as oxygen 12, Japanese Pharmacopoeia Oxygen (manufactured by Shinano Air Water Inc., purity: 99.5% or more) described above or the like can be suitably used, and as helium 11, adjusted helium (manufactured by Nippon Helium Inc.) in which 79.1 to 76.9% of helium and 20.9 to 23.1% of oxygen are mixed beforehand such that the mixed helium and oxygen amount to 100%, or the like can be suitably used.

In a case where the medical gas other than oxygen is helium, the mixing ratio between helium and oxygen is not particularly limited. However, the oxygen concentration after mixing is preferably in the range of 16 to 100%, and more preferably in the range of 21 to 50%. If the oxygen concentration after mixing is less than 21%, suffocation due to low oxygen may occur, and if the oxygen concentration after mixing is more than 50%, in other words, if the helium concentration after mixing is less than 50%, the effect that the gas easily flows through even a narrow airway and ventilation can be improved even in patients having airway stenosis, due to the low density of helium, is significantly weakened.

In the example shown in Fig. 1, a flow controller 14 and a flow meter and oxygen concentration monitor 15 are interposed between mixer 13 and extratracheal tube path 8 in this order, from the side close to mixer 13. Thus, in the present invention, preferably a flow meter for measuring the flow rate of the mixed gas of oxygen and the medical gas other than oxygen, more preferably an oxygen concentration monitor for measuring the oxygen concentration in the mixed gas of oxygen and the medical gas other than oxygen, is connected between the second gas supply device and the extratracheal tube path (or, in the example shown in Fig. 2 described later, the intratracheal tube path). Thus, ventilator 1 in accordance with the present invention is preferably configured to be able to check the flow rate of and the oxygen concentration in the mixed gas of oxygen and the medical gas other than oxygen supplied from the second gas supply device, and control an amount administered to patient 6. As flow controller 14 and flow meter and oxygen concentration monitor 15, any commercially available products can be used as appropriate. Examples of such products which can be suitably used include an area flow meter named RK1202 (manufactured by KOFLOC) as flow controller 14, and Flow Analyzer PF-300 (manufactured by imtmedical (Switzerland)) as flow meter and oxygen concentration monitor 15.

Further, in ventilator 1 in the example shown in Fig. 1, an oxygen concentration sensor 16 is interposed at a location partway along tube path 8a. Thus, in ventilator 1 in accordance with the present invention, an oxygen concentration monitor for measuring the oxygen concentration in the gas containing oxygen on the ventilator side from a location where the second gas supply device is connected is preferably connected between the extratracheal tube path and the first gas supply device. In addition, in the example shown in Fig. 1, a pressure gauge 17 is connected at a location partway along tube path 8d. Oxygen concentration sensor 16 and pressure gauge 17 are connected to a data logger 18, and configured to measure the concentration of oxygen in the gas passing through tube path 8a using oxygen concentration sensor 16, and measure the pressure within tube path 8d using pressure gauge 17.

Preferably, in the present invention, the oxygen concentrations in inhalation gas 5 described above and the mixed gas of oxygen and the medical gas other than oxygen are adjusted as appropriate based on measurement results obtained by flow meter and oxygen concentration monitor 15 and oxygen concentration sensor 16 described above and the like, such that the oxygen concentration in a gas administered to patient 6 (a gas obtained by mixing inhalation gas 5 described above and the mixed gas of oxygen and the medical gas other than oxygen) is preferably in the range of 16 to 100%, and more preferably in the range of 21 to 100%. Although there may be a rare case where the oxygen concentration in the gas administered to patient 6 is 16 to 100%, if the oxygen concentration is less than 21%, suffocation due to low oxygen may occur.

In ventilator 1 in accordance with the present invention, as intratracheal tube path 7 inserted and placed in the trachea of patient 6, a commercially available intratracheal tube used as appropriate for (artificial) ventilation may be used. Specifically, suitable examples of intratracheal tube path 7 include an uncuffed intratracheal tube (siliconized PVC) (manufactured by Smiths Medical (England)), a soft seal cuffed reinforced intratracheal tube (manufactured by Smiths Medical (England)), an uncuffed reinforced intratracheal tube (manufactured by Smiths Medical (England)), a SACETT intratracheal tube (manufactured by Smiths Medical (England)), a soft seal cuffed intratracheal tube (clear PVC) (manufactured by Smiths Medical (England)), a south polar intratracheal tube (soft seal cuff) (manufactured by Smiths Medical (England)), a north polar intratracheal tube (soft seal cuff) (manufactured by Smiths Medical (England)), a south polar intratracheal tube (uncuffed/clear PVC) (manufactured by Smiths Medical (England)), a Blue Line intrabronchial tube (manufactured by Smiths Medical (England)), a TaperGuard intratracheal tube (manufactured by COVIDIEN (the U.S.)), and the like.

Further, Fig. 2 is a view schematically showing a ventilator 31 in another example of the present invention. The ventilator in the example shown in Fig. 2 is the same as ventilator 1 in the example shown in Fig. 1 except for a portion thereof, and parts having the same configurations will be designated by the same reference numerals and the description thereof will not be repeated.

Ventilator 31 in the example shown in Fig. 2 is characterized in that the mixed gas of oxygen and the medical gas other than oxygen from second gas supply device (mixer) 13 is supplied to the intratracheal tube path, instead of a location partway along the extratracheal tube path. By configuring ventilator 31 such that the mixed gas of oxygen and the medical gas other than oxygen is supplied to the intratracheal tube path instead of a location partway along the extratracheal tube path, ventilator 31 can supply the gas more efficiently as is understood when comparison is made between Experimental Example 2 and Experimental Example 3 described later.

When the mixed gas of oxygen and the medical gas other than oxygen is supplied to the intratracheal tube path instead of a location partway along the extratracheal tube path as shown in Fig. 2, an intratracheal tube path having two independent flow paths and configured such that the gas containing oxygen from the first gas supply device flows through one flow path, and the mixed gas of oxygen and the medical gas other than oxygen from the second gas supply device flows through the other flow path is suitably used. As such an intratracheal tube path having two independent flow paths, a commercially available intratracheal tube, specifically, an intratracheal tube shown in the websites of COVIDIEN (http://respiratorysolutions. covidien.com/AirwayManagement/EndotrachealTubes/UncuffedTrachealTubewithMon itoringLumen/tabid/172/Default.aspx), Cardinal Health (http://www.cardinal.com/us/ en/distributedproducts/ASP/43167-025.asp?cat=surgerycenter), HUDSON RCI (http://www.hudsonrci.com/products/product_indiv.asp?catalog=1&PageId=67& prod_cat=21&prod_subcat=38&keywords=), and the like can be suitably used.

Here, Fig. 3 is a view schematically showing an example of the extratracheal tube path and the intratracheal tube path suitably used for ventilator 31 shown in Fig. 2, in which Fig. 3(A) is a perspective view, and Fig. 3(B) is a cross sectional view thereof. For example, Fig. 3 schematically shows a configuration in which a branching member 21 is attached to an end portion of extratracheal tube path 8 to branch extratracheal tube path 8 into a tube path 22 connected to pressure gauge 17, and intratracheal tube path 7. Further, in the example shown in Fig. 3, intratracheal tube path 7 has two independent flow paths 20a, 20b with different sizes, and is configured such that the gas containing oxygen from the first gas supply device through extratracheal tube path 8 flows through larger flow path 20a, whereas smaller flow path 20b has an end portion 23 connected to mixer 13 through flow meter and oxygen concentration monitor 15 and flow controller 14, and the mixed gas of oxygen and the medical gas other than oxygen from the second gas supply device flows through smaller flow path 20b.

### <Experimental Example 1>

First, it was verified beforehand whether or not the helium and oxygen inhalation therapy performed using the method disclosed in NPD 2 (hereinafter referred to as "method A") (Comparative Example 1) is more excellent than the conventional ordinary (artificial) ventilation method, that is, HFO (artificial) ventilation not using a mixed gas of helium and oxygen (hereinafter referred to as "method B") (Comparative Example 2).

Here, Fig. 12 is a view schematically showing a ventilator 101 in a case where the helium and oxygen inhalation therapy is performed using method A. In ventilator 101 shown in Fig. 12, helium 103 and oxygen 104 are mixed by a mixer 105 and supplied as a mixed gas to a main body 102 of the HFO ventilator. Compressed air 106 is also supplied to main body 102. From main body 102, the mixed gas of helium 103 and oxygen 104 is supplied to a respiratory circuit as an inhalation gas 107. Although compressed air 106 is also supplied to main body 102, compressed air 106 is merely consumed for driving main body 102, and is not supplied to the respiratory circuit. Main body 102 is connected to an intratracheal tube path (intratracheal tube) 109 inserted and placed in a trachea of a subject (rabbit) 108, through an extratracheal tube path 110, to form the respiratory circuit. Extratracheal tube path 110 is branched at some locations partway therealong, and includes, other than a tube path 110a for allowing inhalation gas 107 described above to pass therethrough, a tube path 110b for transferring oscillations caused by the HFO from main body 102, a tube path 110c for returning an exhalation gas 111 from subject 108 to main body 102, and a tube path 110d having a patient side thereof connected to intratracheal tube path 109. In the example shown in Fig. 12, extratracheal tube path 110 is configured such that tube path 110b and tube path 110c join at a location partway therealong, further join tube path 110a on a patient side thereof, and connect to tube path 110d. Inhalation gas 107 passes through tube path 110a, then passes through tube path 110d, and flows to intratracheal tube path 109 together with the oscillations caused by the HFO transferred through tube path 110b. On the other hand, exhalation gas 111 from subject 108 passes through tube path 110d and tube path 110c and is returned to main body 102, and thereafter is emitted from main body 102 as an emission gas 112. Further, an oxygen concentration sensor 113 is interposed at a location partway along tube path 110a, and a pressure gauge 114 is connected at a location partway along tube path 110d. Oxygen concentration sensor 113 and pressure gauge 114 are connected to a data logger 115, and configured to measure the concentration of oxygen in the gas passing through tube path 110a using oxygen concentration sensor 113, and measure the pressure within tube path 110d using pressure gauge 114.

Further, Fig. 13 is a view schematically showing a ventilator 201 in a case where it is used for method B. Ventilator 201 in the example shown in Fig. 13 is the same as ventilator 101 in the example shown in Fig. 12 except that oxygen 104 is supplied to main body 102 instead of the mixed gas of helium and oxygen, and parts having the same configurations as those of ventilator 101 in the example shown in Fig. 12 will be designated by the same reference numerals and the description thereof will not be repeated.

In the present experimental example, method B and method A were alternately performed on rabbits as subjects 108, under the HFO (artificial) ventilation. Changes in the partial pressure of oxygen in the arterial blood and the partial pressure of carbon dioxide in the arterial blood of the rabbits were investigated to verify whether method A has an effect equal to or more than that of method B. Details of the experiment will be described below.

In both of methods A and B, as main body 102, a piston-type HFO ventilator named Humming II (manufactured by S. K. I. Net, Inc.) was used. The piston-type HFO ventilator transfers the amplitude of pressure applied by a piston as oscillations of the HFO. Branches were provided at some locations partway along the extratracheal tube path of the ventilator, and pressure gauge 114 for testing (model number: AP-C35, manufactured by Keyence Corporation) was attached thereto. Data logger 115 (LabVIEW8.5 and NI CompactDAQ, manufactured by National Instruments Japan Corporation) was attached to the pressure gauge to record pressure. In the present experimental example, testing conditions were equalized by maintaining the pressure measured with pressure gauge 114 to be constant. Further, as oxygen concentration sensor 113, JKO-25LJII (manufactured by JIKCO Ltd.) was used, and oxygen concentration sensor 113 was also connected to data logger 115, as with pressure gauge 114.

As the mixed gas of helium 103 and oxygen 104 supplied to main body 102 in method A, oxygen (Japanese Pharmacopoeia Oxygen, manufactured by Shinano Air Water Inc., purity: 99.5% or more) and adjusted helium (manufactured by Nippon Helium Inc.) were adjusted by mixer 105 (He+O₂ Blender, manufactured by Air Water Inc.) to have a helium concentration of 50% and an oxygen concentration of 50%. It is noted that the "adjusted helium" is a gas in which 79.1 to 76.9% of helium and 20.9 to 23.1% of oxygen are mixed beforehand such that the mixed helium and oxygen amount to 100%. Further, compressed air 106 was supplied to main body 102 using a compressor (VENTILAIR II, manufactured by Hamilton Medical AG (Switzerland)).

In method A using ventilator 101 shown in Fig. 12, the mixed gas of helium 103 and oxygen 104 having concentrations adjusted by mixer 105 was introduced into an oxygen supply port of main body 102. On this occasion, the oxygen concentration in main body 102 was set to 100%. With this setting, inhalation gas 107 supplied from ventilator 101 to the rabbits as subjects 108 is only the mixed gas of the adjusted helium and oxygen (helium 50%, oxygen 50%). It is noted that the compressed air connected to the ventilator is required for the normal drive of the ventilator, and the compressed air is not contained in the gas administered to the rabbits. In addition, the mixed gas of the adjusted helium and oxygen consumed for driving the ventilator and the mixed gas of the adjusted helium and oxygen supplied to the rabbits were about 36 L/min in total.

In contrast, in method B using ventilator 201 shown in Fig. 13, the concentration of oxygen supplied to main body 102 was set to 50%. With this setting, the gas supplied to the rabbits had an oxygen concentration of 50%.

It is noted that, although the gas administered to the rabbits was not heated and humidified this time, the gas may be administered after being heated and humidified to about 37°C and 100% RH.

As subjects 108, Japanese White Rabbits (model number: Std:JW/CSK, manufactured by Japan SLC, Inc., 11-week old, having a body weight of about 2kg, male) were used. The rabbits were treated before being tested, as described below.

First, an injectable anesthetic was subcutaneously injected to sedate the rabbits. Thereafter, a tracheotomy was performed on each rabbit to attach an intratracheal tube with an internal diameter of 3.5 mm (intratracheal tube path 109) and mount the HFO ventilator. An indwelling needle was placed in a vein, and the injectable anesthetic and a replacement liquid were continuously administered with a syringe pump (Atom Syringe Pump 1235N, manufactured by Atom Medical Corporation).

Subsequently, an indwelling needle (JELCO I. V. Catheter 24G, manufactured by Smiths Medical Japan) was placed in a carotid artery, and a blood collecting port (Interlink I. V. Access System: catheter extension tube, one-adapter line, T-connector type, manufactured by Nippon Becton Dickinson Company, Ltd.) was connected thereto. Blood was collected from the blood collecting port, using an infinitesimal blood collection tube (Terumo Capillary Lithium Heparin VC-C110HL, manufactured by Terumo Corporation). The partial pressure of oxygen in the arterial blood and the partial pressure of carbon dioxide in the arterial blood were measured with a blood gas analysis device (model number: ABL505, manufactured by Radiometer Co., Ltd.).

The experiment was conducted through the procedure described below.
(1) Perform ventilation using method B, and thereafter measure the partial pressure of oxygen in the arterial blood and the partial pressure of carbon dioxide in the arterial blood.
(2) Switch to method A and perform ventilation, and thereafter measure the partial pressure of oxygen in the arterial blood and the partial pressure of carbon dioxide in the arterial blood.
(3) Switch back to method B and perform ventilation, and thereafter measure the partial pressure of oxygen in the arterial blood and the partial pressure of carbon dioxide in the arterial blood.

The above steps (1) to (3) were performed twice on each of four rabbits (rabbits a, b, c, d). It is noted that, in a method of evaluating the results of the present experimental example, the value in method B performed first was set to a ratio of 1, and the value in method A and the value in method B which followed method A were expressed as relative ratios, respectively, in order to eliminate differences in absolute value due to individual differences of the rabbits. Here, a higher value of the partial pressure of oxygen in the arterial blood and a lower value of the partial pressure of carbon dioxide in the arterial blood indicate that ventilation is in a better state. Concerning the partial pressure of oxygen in the arterial blood, when the value in method A was equal to or more than the value in method B, it was determined that the effect by method A was equal to or more than the effect by method B. Similarly, concerning the partial pressure of carbon dioxide in the arterial blood, when the value in method A was equal to or less than the value in method B, it was determined that the effect by method A was equal to or more than the effect by method B. Fig. 14 and Table 1 show the results of the partial pressure of oxygen in the arterial blood, and Fig. 15 and Table 2 show the results of the partial pressure of carbon dioxide in the arterial blood.

**[Table 1]**

| Rabbit Specimen | Raw Data (mmHg) | | | Relative Ratio | | |
|---|---|---|---|---|---|---|
| | Method B | Method A | Method B | Method B | Method A | Method B |
| a-First Time | 147.3 | 192.9 | 148.5 | 1.00 | 1.31 | 1.01 |
| a-Second Time | 148.5 | 168.7 | 136.5 | | 1.14 | 0.92 |
| b-First Time | 140.6 | 178.8 | 144.3 | | 1.27 | 1.03 |
| b-Second Time | 144.3 | 172.2 | 117.6 | | 1.19 | 0.81 |
| c-First Time | 136.2 | 188.5 | 127.8 | | 1.38 | 0.94 |
| c-Second Time | 127.8 | 175.8 | 140.7 | | 1.38 | 1.10 |
| d-First Time | 145.9 | 193.8 | 138.3 | | 1.33 | 0.95 |
| d-Second Time | 138.3 | 181.3 | 120.3 | | 1.31 | 0.87 |

**[Table 2]**

| Rabbit Specimen | Raw Data (mmHg) | | | Relative Ratio | | |
|---|---|---|---|---|---|---|
| | Method B | Method A | Method B | Method B | Method A | Method B |
| a-First Time | 82.6 | 72.2 | 93.8 | 1.00 | 0.87 | 1.14 |
| a-Second Time | 93.8 | 85.4 | 109.4 | | 0.91 | 1.17 |
| b-First Time | 109.3 | 105.1 | 125.2 | | 0.96 | 1.15 |
| b-Second Time | 125.2 | 112.0 | 132.7 | | 0.89 | 1.06 |
| c-First Time | 111.9 | 99.6 | 107.4 | | 0.89 | 0.96 |
| c-Second Time | 107.4 | 95.7 | 102.0 | | 0.89 | 0.95 |
| d-First Time | 97.5 | 78.3 | 101.2 | | 0.80 | 1.04 |
| d-Second Time | 101.2 | 90.2 | 112.6 | | 0.89 | 1.11 |

As can be seen from Tables 1, 2 and Figs. 14, 15, method A improved the partial pressure of oxygen in the arterial blood and the partial pressure of carbon dioxide in the arterial blood, when compared with method B. Therefore, it was also confirmed in the present test that the method of administering the mixed gas of helium and oxygen by the HFO is more effective than the conventional ordinary (artificial) ventilation method.

### <Experimental Example 2>

HFO (artificial) ventilation which supplied a mixed gas of helium and oxygen from a branch at a location partway along extratracheal tube path 8 using ventilator 1 in accordance with the present invention in the example shown in Fig. 1 (hereinafter referred to as "method C") (Example 1), and method A described above (Comparative Example 1) were alternately performed, to investigate changes in the partial pressure of oxygen in the arterial blood and the partial pressure of carbon dioxide in the arterial blood of rabbits, and verify whether method C has an effect equal to or more than that of method A.

In method C, the concentration of oxygen supplied to main body 2 was set to 50%, as in method B described above. In method C, adjusted helium 11 and oxygen 12 identical to those described above for method A were used and adjusted by mixer 13 to have a helium concentration of 50% and an oxygen concentration of 50%. Thereafter, the flow rate was adjusted by flow controller 14 (area flow meter named RK1202, manufactured by KOFLOC), and further, the flow rate and the oxygen concentration were checked by flow meter and oxygen concentration monitor 15 (Flow Analyzer PF-300, manufactured by imtmedical (Switzerland)). Then, the mixed gas was supplied from the branch at a location partway along extratracheal tube path 8. Thus, the gas supplied to the rabbits was set to have an oxygen concentration of 50%.

First, an administration amount in method C was set through the procedure described below.
(1) Perform ventilation using method A, and thereafter measure the partial pressure of oxygen in the arterial blood and the partial pressure of carbon dioxide in the arterial blood.
(2) Switch to method C and perform ventilation, and thereafter measure the partial pressure of oxygen in the arterial blood and the partial pressure of carbon dioxide in the arterial blood. Set the administration flow rate of the mixed gas of helium and oxygen to 0.50 L/min.
(3) Switch back to method A and perform ventilation, and thereafter measure the partial pressure of oxygen in the arterial blood and the partial pressure of carbon dioxide in the arterial blood.
(4) Switch to method C and perform ventilation, and thereafter measure the partial pressure of oxygen in the arterial blood and the partial pressure of carbon dioxide in the arterial blood. Set the administration flow rate of the mixed gas of helium and oxygen to 1.00 L/min.

Thereafter, the procedure of using method A, method C with the administration amount being set to 1.50 L/min, and then method A ... was repeatedly performed with the administration amount being incremented by 0.50 L/min, until the administration amount in method C reached 4.00 L/min. It is noted that a rabbit g of three rabbits described later was used to set the administration amount in method C. Figs. 4 and 5 and Table 3 show the results. As a result, the administration amount in method C was determined to 2.00 L/min.

**[Table 3]**

| Rabbit g | Method A | MethodC 0.50L/min | Method A | MethodC 1.00L/min | Method A | MethodC 1.50L/min | Method A | MethodC 2.00L/min | Method A | MethodC 2.50L/min | Method A | MethodC 3.00L/min | Method A | MethodC 3.50L/min | Method A | MethodC 4.00L/min | Method A |
|---|---|---|---|---|---|---|---|---|---|---|---|---|---|---|---|---|---|
| Partial Pressure of Oxygen in Arterial Blood (mmHg) | 203.0 | 180.0 | 207.2 | 188.8 | 189.5 | 185.7 | 201.7 | 225.1 | 215.2 | 238.5 | 218.9 | 238.7 | 214.1 | 244.2 | 216.1 | 249.0 | 230.1 |
| Partial Pressure of Carbon Dioxide in Arterial Blood (mmHg) | 67.4 | 73.0 | 69.3 | 72.4 | 75.2 | 74.5 | 79.6 | 72.2 | 75.8 | 69.4 | 72.1 | 59.2 | 66.7 | 55.6 | 60.3 | 46.9 | 57.5 |

Next, whether method C has an effect equal to or more than that of method A was examined through the procedure described below.
(5) Perform ventilation using method A, and thereafter measure the partial pressure of oxygen in the arterial blood and the partial pressure of carbon dioxide in the arterial blood.
(6) Switch to method C and perform ventilation, and thereafter measure the partial pressure of oxygen in the arterial blood and the partial pressure of carbon dioxide in the arterial blood.
(7) Switch back to method A and perform ventilation, and thereafter measure the partial pressure of oxygen in the arterial blood and the partial pressure of carbon dioxide in the arterial blood.

The above steps (5) to (7) were performed twice on each of the three rabbits (rabbits e, f, g). Figs. 6, 7 and Tables 4, 5 show the results.

It is noted that, also in a method of evaluating the results of Experimental Example 2, the value in method A performed first was set to a ratio of 1, and the value in method C and the value in method A which followed method C were expressed as relative ratios, respectively, in order to eliminate differences in absolute value due to individual differences of the rabbits, as in Experimental Example 1. Concerning the partial pressure of oxygen in the arterial blood, when the value in method C was equal to or more than the value in method A, it was determined that the effect by method C was equal to or more than the effect by method A. Similarly, concerning the partial pressure of carbon dioxide in the arterial blood, when the value in method C was equal to or less than the value in method A, it was determined that the effect by method C was equal to or more than the effect by method A.

**[Table 4]**

| Rabbit Specimen | Raw Data (mmHg) | | | Relative Ratio | | |
|---|---|---|---|---|---|---|
| | Method A | Method C | Method A | Method A | Method C | Method A |
| e-First Time | 240.9 | 254.4 | 233.5 | 1.00 | 1.06 | 0.97 |
| e-Second Time | 233.5 | 247.1 | 210.2 | | 1.06 | 0.90 |
| f-First Time | 185.4 | 202.5 | 190.0 | | 1.09 | 1.02 |
| f-Second Time | 207.6 | 216.5 | 197.4 | | 1.04 | 0.95 |
| g-First Time | 192.5 | 207.5 | 194.8 | | 1.08 | 1.01 |
| g-Second Time | 194.8 | 207.9 | 201.1 | | 1.07 | 1.03 |

**[Table 5]**

| Rabbit Specimen | Raw Data (mmHg) | | | Relative Ratio | | |
|---|---|---|---|---|---|---|
| | Method A | Method C | Method A | Method A | Method C | Method A |
| e-First Time | 46.7 | 42.7 | 46.2 | 1.00 | 0.91 | 0.99 |
| e-Second Time | 46.2 | 41.3 | 44.5 | | 0.89 | 0.96 |
| f-First Time | 65.4 | 62.3 | 67.0 | | 0.95 | 1.02 |
| f-Second Time | 71.0 | 64.3 | 68.0 | | 0.91 | 0.96 |
| g-First Time | 68.5 | 62.3 | 66.8 | | 0.91 | 0.98 |
| g-Second Time | 66.8 | 61.9 | 68.0 | | 0.93 | 1.02 |

As can be seen from the results shown in Figs. 6, 7 and Tables 4, 5, method C was able to have an effect equal to or more than that of method A. That is, it was found that an amount of the gas of helium and oxygen of 2.00 L/min as set in test C is enough to obtain an equal effect under these conditions, without having to set the amount of the gas of helium and oxygen to 36 L/min as in test A.

It is noted that, although the gas administered to the rabbits was not heated and humidified this time, the gas may be administered after being heated and humidified to about 37°C and 100% RH

### <Experimental Example 3>

HFO (artificial) ventilation which supplied a mixed gas of helium and oxygen from a leading end of intratracheal tube path 7 using ventilator 31 in accordance with the present invention in the example shown in Fig. 2 (hereinafter referred to as "method D") (Example 2), and method A described above (Comparative Example 1) were alternately performed, to investigate changes in the partial pressure of oxygen in the arterial blood and the partial pressure of carbon dioxide in the arterial blood of rabbits, and verify whether method D has an effect equal to or more than that of method A.

In method D, the concentration of oxygen supplied to main body 2 was set to 50%, as in method B described above. In method D, adjusted helium 11 and oxygen 12 identical to those described above for method A were used and adjusted by mixer 13 to have a helium concentration of 50% and an oxygen concentration of 50%. Thereafter, the flow rate was adjusted by flow controller 14 (area flow meter named RK1202, manufactured by KOFLOC), and further, the flow rate and the oxygen concentration were checked by flow meter and oxygen concentration monitor 15 (Flow Analyzer PF-300, manufactured by imtmedical (Switzerland)). Then, the mixed gas was supplied from the leading end of intratracheal tube path 7. Thus, the gas supplied to the rabbits was set to have an oxygen concentration of 50%.

In method D, as intratracheal tube path 7, an intratracheal tube shown in the website of COVIDIEN (http://respiratorysolutions.covidien.com/AirwayManagement/ EndotrachealTubes/UncuffedTrachealTubewithMonitoringLumen/tabid/172/Default.as px) was used in order to supply the mixed gas of helium and oxygen from the leading end of intratracheal tube path 7. Further, in method D, an intratracheal tube shown in the websites of Cardinal Health (http://www.cardinal.com/us/en/distributedproducts/ ASP/43167-025.asp?cat=surgerycenter) and HUDSON RCI (http://www.hudsonrci. com/products/product_indiv.asp?catalog=1&PageId=67&prod_cat=21&prod_subcat=3 8&keywords=) can also be suitably used.

First, an administration amount in method D was set through the procedure described below.
(1) Perform ventilation using method A, and thereafter measure the partial pressure of oxygen in the arterial blood and the partial pressure of carbon dioxide in the arterial blood.
(2) Switch to method D and perform ventilation, and thereafter measure the partial pressure of oxygen in the arterial blood and the partial pressure of carbon dioxide in the arterial blood. Set the administration flow rate of the mixed gas of helium and oxygen to 0.10 L/min.
(3) Switch back to method A and perform ventilation, and thereafter measure the partial pressure of oxygen in the arterial blood and the partial pressure of carbon dioxide in the arterial blood.
(4) Switch to method D and perform ventilation, and thereafter measure the partial pressure of oxygen in the arterial blood and the partial pressure of carbon dioxide in the arterial blood. Set the administration flow rate of the mixed gas of helium and oxygen to 0.20 L/min.

Thereafter, the procedure of using method A, method D with the administration amount being incremented by 0.10 L/min, and then method A ... was repeatedly performed until the administration amount in method D reached 0.50 L/min. Figs. 8, 9 and Tables 6, 7 show the results. It is noted that a rabbit i of three rabbits described later and another rabbit 1 were used to set the administration amount in method D. Rabbit 1 was used only when the administration flow rate of the mixed gas of helium and oxygen was set to 0.20 L/min. Table 6 shows changes in the partial pressure of oxygen in the arterial blood of the rabbits when the administration amount in method D was set, and Table 7 shows changes in the partial pressure of carbon dioxide in the arterial blood of the rabbits when the administration amount in method D was set. As a result, the administration amount in method D was determined to 0.30 L/min.

**[Table 6]**

| Rabbit Specimen | Method A | Method D 0.10L/min | Method A | Method D 0.20L/min | Method A | Method D 0.30L/min | Method A | Method D 0.40L/min | Method A | Method D 0.50L/min | Method A |
|---|---|---|---|---|---|---|---|---|---|---|---|
| i | 215.4 | 187.1 | 217.1 | 208.9 | 209.2 | 210.8 | 202.6 | 217.9 | 205.6 | 210.8 | 208.0 |
| 1-First Time | | | 256.4 | 248.1 | 259.7 | | | | | | |
| 1-Second Time | | | 259.7 | 250.5 | | | | | | | |

**[Table 7]**

| Rabbit Specimen | Method A | Method D 0.10L/min | Method A | Method D 0.20L/min | Method A | Method D 0.30L/min | Method A | Method D 0.40L/min | Method A | Method D 0.50L/min | Method A |
|---|---|---|---|---|---|---|---|---|---|---|---|
| i | 72.9 | 76.8 | 77.3 | 68.7 | 75.2 | 63.5 | 75.8 | 59.0 | 84.7 | 61.1 | 76.6 |
| 1-First Time | | | 52.6 | 50.1 | 51.1 | | | | | | |
| 1-Second Time | | | 51.1 | 58.5 | | | | | | | |

Next, whether method D has an effect equal to or more than that of method A was examined through the procedure described below.
(5) Perform ventilation using method A, and thereafter measure the partial pressure of oxygen in the arterial blood and the partial pressure of carbon dioxide in the arterial blood.
(6) Switch to method D and perform ventilation, and thereafter measure the partial pressure of oxygen in the arterial blood and the partial pressure of carbon dioxide in the arterial blood.
(7) Switch back to method A and perform ventilation, and thereafter measure the partial pressure of oxygen in the arterial blood and the partial pressure of carbon dioxide in the arterial blood.

The above steps (5) to (7) were performed once to three times on the three rabbits (rabbits h, i, j). Figs. 10, 11 and Tables 8, 9 show the results.

It is noted that, also in a method of evaluating the results of Experimental Example 3, the value in method A performed first was set to a ratio of 1, and the value in method D and the value in method A which followed method D were expressed as relative ratios, respectively, in order to eliminate differences in absolute value due to individual differences of the rabbits, as in Experimental Example 1. Further, also in Experimental Example 3, the partial pressure of oxygen in the arterial blood and the partial pressure of carbon dioxide in the arterial blood were evaluated by sandwiching method D between methods A as a test order to eliminate physical changes in the rabbits. It is noted that a higher value of the partial pressure of oxygen in the arterial blood and a lower value of the partial pressure of carbon dioxide in the arterial blood indicate that ventilation is in a better state. Concerning the partial pressure of oxygen in the arterial blood, when the value in method D was equal to or more than the value in method A, it was determined that the effect by method D was equal to or more than the effect by method A. Similarly, concerning the partial pressure of carbon dioxide in the arterial blood, when the value in method D was equal to or less than the value in method A, it was determined that the effect by method D was equal to or more than the effect by method A.

**[Table 8]**

| Rabbit Specimen | Raw Data (mmHg) | | | Relative Ratio | | |
|---|---|---|---|---|---|---|
| | Method A | Method D | Method A | Method A | Method D | Method A |
| h-First Time | 207.3 | 231.6 | 219.7 | 1.00 | 1.12 | 1.06 |
| i-First Time | 205.9 | 216.2 | 206.3 | | 1.05 | 1.00 |
| i-Second Time | 206.3 | 206.6 | 208.9 | | 1.00 | 1.01 |
| i-Third Time | 209.2 | 210.8 | 202.6 | | 1.01 | 0.97 |
| j-First Time | 187.6 | 206.4 | 212.4 | | 1.10 | 1.13 |
| j-Second Time | 201.9 | 196.5 | 184.6 | | 0.97 | 0.91 |

**[Table 9]**

| Rabbit Specimen | Raw Data (mmHg) | | | Relative Ratio | | |
|---|---|---|---|---|---|---|
| | Method A | Method D | Method A | Method A | Method D | Method A |
| h-First Time | 59.9 | 48.4 | 56.3 | 1.00 | 0.81 | 0.94 |
| i-First Time | 83.6 | 69.1 | 79.1 | | 0.83 | 0.95 |
| i-Second Time | 79.1 | 68.1 | 78.2 | | 0.86 | 0.99 |
| i-Third Time | 75.2 | 63.5 | 75.8 | | 0.84 | 1.01 |
| j-First Time | 71.6 | 64.1 | 70.4 | | 0.90 | 0.98 |
| j-Second Time | 73.7 | 68.6 | 73.6 | | 0.93 | 1.00 |

As can be seen from the results shown in Figs. 10, 11 and Tables 8, 9, method D was also able to have an effect equal to or more than that of method A. That is, it was found that an amount of the gas of helium and oxygen of 0.30 L/min as set in test D is enough to obtain an equal effect under these conditions, without having to set the amount of the gas of helium and oxygen to 36 L/min as in test A.

It is noted that, although the gas administered to the rabbits was not heated and humidified this time, the gas may be administered after being heated and humidified to about 37°C and 100% RH

The above results indicate that, when compared with the method of administering the mixed gas of helium and oxygen by the HFO in the (artificial) ventilation therapy (method A), method C which supplied the mixed gas of helium and oxygen from a location partway along the extratracheal tube path was able to reduce the consumption amount of helium to (2.00L/min)/(36L/min)=5.6%, and method D which supplied the mixed gas of helium and oxygen to the intratracheal tube path was able to reduce the consumption amount of helium to (0.30L/min)/(36L/min)=0.83%. Thus, with one helium cylinder having a capacity of 7000L, treatment can be continued, for example, for 58.3 hours in method C, and for 388.9 hours in method D, and the frequency of replacing the cylinders can also be significantly reduced, leading to a reduction of the burden on medical staff. Accordingly, in practice, the helium and oxygen inhalation therapy can be performed as a practical treatment method.

Such an effect is considered to be exerted on the reduction of pressure loss in the intratracheal tube by helium. In the method disclosed in NPD 2 shown in Fig. 12 (method A), not only the intratracheal tube path but also the extratracheal tube path and the inside of the main body of the HFO ventilator are filled with the mixed gas of helium and oxygen. Thus, helium and oxygen are also used in the gas for driving the HFO ventilator. In contrast, in the present invention, the mixed gas of helium and oxygen is supplied only to the vicinity of the intratracheal tube path where the effect appears to be exerted, which is considered as the reason why the consumption amount of the mixed gas of helium and oxygen can be reduced without deteriorating the effect of improving the efficiency of (artificial) ventilation using the mixed gas of helium and oxygen.

It should be understood that the embodiments disclosed herein are illustrative and non-restrictive in every respect. The scope of the present invention is defined by the scope of the claims, rather than the description above, and is intended to include any modifications within the scope and meaning equivalent to the scope of the claims.

### REFERENCE SIGNS LIST

1, 31: ventilator; 2: first gas supply device (main body); 3: oxygen; 4: compressed air; 5: inhalation gas; 6: patient (subject); 7: intratracheal tube path; 8: extratracheal tube path; 9: exhalation gas; 10: emission gas; 11: helium; 12: oxygen; 13: second gas supply device (mixer); 14: flow controller; 15: flow meter and oxygen concentration monitor; 16: oxygen concentration sensor; 17: pressure gauge; 18: data logger.

## Claims

1. A ventilator (1, 31) for supplying a mixed gas of oxygen and a medical gas other than oxygen to a patient as an inhalation gas (5), comprising:
a first gas supply device (2) for supplying a gas containing oxygen, wherein the first gas supply device (2) supplies the gas containing oxygen by high frequency oscillation;
an intratracheal tube path (7) inserted and placed in a trachea of the patient;
an extratracheal tube path (8) for connecting the first gas supply device (2) with the intratracheal tube path (7) and guiding the gas containing oxygen from the first gas supply device (2) to the patient; and
a second gas supply device (13) connected to the extratracheal tube path (8) or the intratracheal tube path (7) for supplying the mixed gas of oxygen and the medical gas other than oxygen to the extratracheal tube path (8) or the intratracheal tube path (7).

2. The ventilator (1, 31) according to claim 1, wherein the medical gas other than oxygen is helium.

3. The ventilator (1, 31) according to claim 1, wherein a flow meter for measuring a flow rate of the mixed gas of oxygen and the medical gas other than oxygen is connected between the second gas supply device (13) and the intratracheal tube path (7) or the extratracheal tube path (8).

4. The ventilator (1, 31) according to claim 1, wherein an oxygen concentration monitor for measuring an oxygen concentration in the mixed gas of oxygen and the medical gas other than oxygen is connected between the second gas supply device (13) and the intratracheal tube path (7) or the extratracheal tube path (8).

5. The ventilator (1, 31) according to claim 1, wherein an oxygen concentration monitor for measuring an oxygen concentration in the gas containing oxygen on a ventilator side from a location where the second gas supply device (13) is connected is connected between the extratracheal tube path (8) and the first gas supply device (2).

6. The ventilator (1, 31) according to claim 1, wherein the intratracheal tube path (7) has two independent flow paths, and is configured such that the gas containing oxygen from the first gas supply device (2) flows through one flow path, and the mixed gas of oxygen and the medical gas other than oxygen from the second gas supply device (13) flows through the other flow path.

7. The ventilator (1, 31) according to claim 1, wherein the gas containing oxygen from the first gas supply device (2) contains compressed air.

8. The ventilator (1, 31) according to claim 1, wherein the first gas supply device (2) is configured to emit an exhalation gas from the patient passing through the intratracheal tube path (7) and the extratracheal tube path (8).

## Patentansprüche

1. Belüfter (1, 31) zum Zuführen eines Mischgases von Sauerstoff und einem medizinischen Gas außer Sauerstoff zu einem Patienten als Atemgas (5), welcher aufweist:
eine erste Gaszufuhrvorrichtung (2) zum Zuführen eines sauerstoffhaltigen Gases, wobei die erste Gaszufuhrvorrichtung (2) das sauerstoffhaltige Gas durch Hochfrequenzoszillation zuführt;
einen Intratrachealrohrweg (7), der in eine Trachea des Patienten eingesetzt und dort platziert wird; und
einen Extratrachealrohrweg (8) zum Verbinden der ersten Gaszufuhrvorrichtung (2) mit dem Intratrachealrohrweg (7) und Leiten des sauerstoffhaltigen Gases von der ersten Gaszufuhrvorrichtung (2) zu dem Patienten; und
eine zweite Gaszufuhrvorrichtung (13), die mit dem Extratrachealrohrweg (8) oder dem Intratrachealrohrweg (7) verbunden ist, um das Mischgas von Sauerstoff und dem medizinischen Gas außer Sauerstoff dem Extratrachealrohrweg (8) oder dem Intratrachealrohrweg (7) zuzuführen.

2. Der Belüfter (1, 31) nach Anspruch 1, wobei das medizinische Gas außer Sauerstoff Helium ist.

3. Der Belüfter (1, 31) nach Anspruch 1, wobei ein Strömungsmesser zum Messen einer Strömungsrate des Mischgases von Sauerstoff und dem medizinischen Gas außer Sauerstoff zwischen der zweiten Gaszufuhrvorrichtung (13) und dem Intratrachealrohrweg (7) oder dem Extratrachealrohrweg (8) angeschlossen ist.

4. Der Belüfter (1, 31) nach Anspruch 1, wobei ein Sauerstoffkonzentrationsmonitor zum Messen einer Sauerstoffkonzentration in dem Mischgas von Sauerstoff und dem medizinischen Gas außer Sauerstoff zwischen der zweiten Gaszufuhrvorrichtung (13) und dem Intratrachealrohrweg (7) oder dem Extratrachealrohrweg (8) angeschlossen ist.

5. Der Belüfter (1, 31) nach Anspruch 1, wobei ein Sauerstoffkonzentrationsmonitor zum Messen einer Sauerstoffkonzentration in dem sauerstoffhaltigen Gas an einer Belüfterseite von einem Ort, wo die zweite Gaszufuhrvorrichtung (13) angeschlossen ist, zwischen dem Extratrachealrohrweg (8) und der ersten Gaszufuhrvorrichtung (2) angeschlossen ist.

6. Der Belüfter (1, 31) nach Anspruch 1, wobei der Intratrachealrohrweg (7) zwei unabhängige Strömungswege aufweist und derart konfiguriert ist, dass das sauerstoffhaltige Gas von der ersten Gaszufuhrvorrichtung (2) durch den einen Strömungsweg fließt und das Mischgas von Sauerstoff und dem medizinischen Gas außer Sauerstoff von der zweiten Gaszufuhrvorrichtung (13) durch den anderen Strömungsweg fließt.

7. Der Belüfter (1, 31) nach Anspruch 1, wobei das sauerstoffhaltige Gas von der ersten Gaszufuhrvorrichtung (2) Druckluft enthält.

8. Der Belüfter (1, 31) nach Anspruch 1, wobei die erste Gaszufuhrvorrichtung (2) konfiguriert ist, um Exhalationsgas von dem Patienten abzugeben, das durch den Intratrachealrohrweg (7) und den Extratrachealrohrweg (8) hindurchtritt.

## Revendications

1. Ventilateur (1, 31) pour fournir à un patient en tant que gaz d'inspiration (5) un gaz mixte d'oxygène et d'un gaz médical autre que l'oxygène, comprenant :
un premier dispositif d'alimentation en gaz (2) pour fournir un gaz contenant de l'oxygène, où le premier dispositif d'alimentation en gaz (2) fournit le gaz contenant de l'oxygène par une oscillation haute fréquence ;
un trajet de tube endotrachéal (7) inséré et placé dans la trachée du patient ;
un trajet de tube extra-trachéal (8) pour relier le premier dispositif d'alimentation en gaz (2) au trajet de tube endotrachéal (7) et acheminer vers le patient le gaz contenant de l'oxygène provenant du premier dispositif d'alimentation en gaz (2) ; et
un deuxième dispositif d'alimentation en gaz (13) relié au trajet de tube extra-trachéal (8) ou au trajet de tube endotrachéal (7) pour fournir le gaz mixte d'oxygène et du gaz médical autre que l'oxygène au trajet de tube extra-trachéal (8) ou au trajet de tube endotrachéal (7).

2. Ventilateur (1, 31) selon la revendication 1, dans lequel le gaz médical autre que l'oxygène est l'hélium.

3. Ventilateur (1, 31) selon la revendication 1, dans lequel un débitmètre pour mesurer un débit du gaz mixte d'oxygène et du gaz médical autre que l'oxygène est relié entre le deuxième dispositif d'alimentation en gaz (13) et le trajet de tube endotrachéal (7) ou le trajet de tube extra-trachéal (8).

4. Ventilateur (1, 31) selon la revendication 1, dans lequel un dispositif de surveillance de la concentration en oxygène destiné à mesurer une concentration en oxygène dans le gaz mixte d'oxygène et du gaz médical autre que l'oxygène est relié entre le deuxième dispositif d'alimentation en gaz (13) et le trajet de tube endotrachéal (7) ou le trajet de tube extra-trachéal (8).

5. Ventilateur (1, 31) selon la revendication 1, dans lequel un dispositif de surveillance de la concentration en oxygène destiné à mesurer une concentration en oxygène dans le gaz contenant de l'oxygène sur un côté ventilateur à partir d'un emplacement où le deuxième dispositif d'alimentation en gaz (13) est relié, est relié entre le trajet de tube extra-trachéal (8) et le premier dispositif d'alimentation en gaz (2).

6. Ventilateur (1, 31) selon la revendication 1, dans lequel le trajet de tube endotrachéal (7) a deux trajets d'écoulement indépendants, et est configuré de sorte que le gaz contenant de l'oxygène provenant du premier dispositif d'alimentation en gaz (2) s'écoule à travers un trajet d'écoulement, et le gaz mixte d'oxygène et du gaz médical autre que l'oxygène provenant du deuxième dispositif d'alimentation en gaz (13) s'écoule à travers l'autre trajet d'écoulement.

7. Ventilateur (1, 31) selon la revendication 1, dans lequel le gaz contenant de l'oxygène provenant du premier dispositif d'alimentation en gaz (2) contient de l'air comprimé.

8. Ventilateur (1, 31) selon la revendication 1, dans lequel le premier dispositif d'alimentation en gaz (2) est configuré pour émettre un gaz d'expiration du patient passant à travers le trajet de tube endotrachéal (7) et le trajet de tube extra-trachéal (8).
